# EUROPÄISCHE PATENTSCHRIFT

(11) **EP 3 103 393 B1**
(45) Veröffentlichungstag und Bekanntmachung des Hinweises auf die Patenterteilung: **30.08.2017**
(21) Anmeldenummer: 16172071.9
(22) Anmeldetag: 31.05.2016
(51) Int. Cl.: A61B 5/1473, A61B 5/1495, A61B 5/145, A61B 5/00, G01N 7/10, G01N 15/06, G01N 33/49, G01N 15/00

(54) **QUEREMPFINDLICHKEITSKOMPENSIERTER BIOSENSOR**
CROSS-SENSITIVITY COMPENSATED BIOSENSOR
BIO-CAPTEUR COMPENSE A SENSIBILITE CROISEE

(30) Priorität: 01.06.2015 DE 102015108644
(43) Veröffentlichungstag der Anmeldung: 14.12.2016
(73) Patentinhaber: BIOTRONIK SE & Co. KG, 12359 Berlin (DE)
(72) Erfinder: Ebert, Henning, 10317 Berlin (DE); Bunge, Andreas, 04105 Leipzig (DE)
(74) Vertreter: Galander, Marcus

(56) Entgegenhaltungen:
- US-A1- 2009 241 681
- US-A1- 2011 124 985
- US-B1- 6 546 268

## Beschreibung

Die Erfindung betrifft einen Querempfindlichkeitskompensierten Biosensor nach den Oberbegriffen des Anspruchs 1.

Als Biosensor wird im Folgenden ein Sensor bezeichnet der zum Nachweis oder zur Konzentrationsbestimmung von Analyten in Medien innerhalb oder außerhalb des menschlichen oder tierischen Körpers dient. Ein Medium das ggf einen Analyten enthält der nachgewiesen werden soll oder dessen Konzentration bestimmt werden soll wird als Testmedium bezeichnet.

Zur Realisierung neuartiger Sensoren für die Konzentrationsbestimmung oder den Nachweis von Substanzen können Polymernetzwerke verwendet werden. Polymernetzwerke sind Polymere, deren Moleküle chemisch (z.B. durch kovalente oder ionische Bindungen) oder physikalisch (z.B. durch Verschlaufen der Polymerketten) zu einem dreidimensionalen Netzwerk verknüpft sind. In Gegenwart eines günstigen Lösungsmittels quillt ein solches Netzwerk, indem es dieses aufnimmt. Man bezeichnet das gequollene Netzwerk dann auch als Gel oder auch als Polymergel. Sofern Wasser das Quellmittel ist, spricht man auch von einem Hydrogel. Als Spezialfall der Gele tragen "Smarte" Gele (Bei Wasser als Quellmittel "Smarte" Hydrogele) die Fähigkeit, unter bestimmten Voraussetzungen selektiv auf Änderungen von Umgebungsgrößen mit Volumenänderungen zu reagieren. Sensitivitäten sind insbesondere gegenüber Temperatur, pH-Wert, Ionen- oder Stoffkonzentrationen erzielbar. Somit können solche Polymernetzwerke bei Änderung der Konzentration einer Substanz beispielsweise im Blut mit einer starken Volumenänderung durch Wasseraufnahme bzw. Wasserabgabe reagieren. Eingebracht in ein definiertes Volumen kann man die konzentrationsabhängige Volumenänderung in eine konzentrationsabhängige Druckänderung übersetzen. Dies ist insbesondere bei Sensoren relevant, mittels derer man die Konzentrationen bestimmter Substanzen (z.B. sog. Biomarker z.B. im Blut) ermitteln kann.

Für die Erzeugung der "smarten" Gele werden häufig Basismaterialien eingesetzt, die neben der Sensitivität für die Substanzen, zu deren Nachweis oder Konzentrationsbestimmung sie erzeugt werden, zusätzlich eine starke Abhängigkeit von anderen Ionen- oder Stoffkonzentrationen und/oder der Temperatur und/oder vom pH-Wert zeigen, d.h. diese Gele können ihren Lösungsmittelgehalt (resp. Volumen) in Abhängigkeit von Ionen- oder Stoffkonzentrationen und/oder von der Temperatur und/oder des pH-Wert ändern. Die Volumen- bzw. Druckänderung des Gels sind somit abhängig von der Konzentration der Substanz, der eigentlichen Sensormessgröße, so wie der Konzentration anderer Ionen- und/oder Stoffkonzentrationen und/oder der Temperatur und/oder dem pH-Wert. Es hat sich herausgestellt, dass die Volumen- bzw. Quelldruckänderung bei Variationen der Temperatur und/oder des pH-Werts deutlich größer sind als durch die Änderung der eigentlichen Sensormessgröße - z. B. der Konzentration des Blutbestandteils beim Einsatz von "smarten" Hydrogelen.

Die Empfindlichkeit eines Sensors auf andere Größen als die zu messende Größe wird als Querempfindlichkeit bezeichnet. Die zu messende Größe ist die Messgröße oder auch Sensormessgröße. Eine Größe, die nicht Messgröße ist, jedoch die von der Messeinrichtung gelieferte Information über den Messwert beeinflusst, heißt Einflussgröße. Die Einflussgröße bewirkt, dass sich der Messwert allein schon dadurch ändert, dass sich die Einflussgröße ändert.

Die US 2010/0056888 A1 offenbart einen implantierbaren Biosensor, bei dem ein Hydrogel zum Nachweis eines Analyten eingesetzt werden kann. Ein Hydrogel kann bei Anwesenheit eines geeigneten Analyten eine starke Volumenänderung durchführen. Zur Messung einer Konzentration eines Analyten werden zwei Drucksensoren eingesetzt. Einer misst den vom Analyten veränderbaren Druck in einer Messkammer, ein anderer misst den Druck außerhalb der Messkammer. Aus der Druckdifferenz wird der Gehalt des Analyten in der Messkammer bestimmt. Die beiden Druckwerte können ebenso zur Temperaturkompensation dienen.

Die US 2009/0275815 A1 offenbart ein System mit einem Biosensor zum Messen der Konzentration eines Analyten und einem Temperatursensor, der die Temperatur in einer Region, die möglichst eng benachbart zum Biosensor ist, erfasst. Das Ausgangssignal des Biosensors wird mit der gemessenen Temperatur korrigiert, um die gemessene Analytkonzentration zu bestimmen.

Die hohen technischen Anforderungen an die Sensorik, wie Messbereiche und Auflösung des Druck- und Temperatursensors, können zum hochpräzisen Nachweis medizinisch relevanter Konzentrationsänderungen im Blut insbesondere in einem transportablen bzw. implantierbaren System nur schwer realisiert werden. Dokument US 2009/241681 offenbart einen Biosensor gemäß dem Oberbegriff des Anspruchs 1. Der Erfindung liegt die Aufgabe zugrunde, einen verbesserten querempfindlichkeitskompensierten Biosensor zu schaffen, der einen zuverlässigen und präzisen Nachweis von Analyten erlaubt.

Die Aufgabe wird erfindungsgemäß durch die Merkmale des unabhängigen Anspruchs gelöst. Günstige Ausgestaltungen und Vorteile der Erfindung ergeben sich aus den weiteren Ansprüchen, der Beschreibung und der Zeichnung.

Es wird ein Biosensor vorgeschlagen, mit einem für wenigstens einen Analyten sensiblen Sensormaterial in einem mit einem Drucksensor gekoppelten Druckmessraum, wobei ein im Druckmessraum herrschender Druck durch den Drucksensor bestimmbar ist. Das Sensormaterial ist im Druckmessraum mit einem Kompensationsmaterial gekoppelt, welches eine querempfindlichkeitsbedingte Volumenänderung des Sensormaterials zumindest teilweise kompensiert.

Unter einem Drucksensor ist jede Einrichtung zu verstehen, die zur Bestimmung des im Druckmessraum herrschenden Drucks geeignet ist.

Vorzugsweise handelt es sich bei dem Biosensor um einen in den menschlichen oder tierischen Körper implantierbaren Biosensor.

Vorzugsweise handelt es sich bei dem für wenigstens einen Analyten sensiblen Sensormaterial um ein für wenigstens einen Analyten sensibles Polymergel, oder Hydrogel oder "smartes" Hydrogel.

Der Sensor ist zur Messung in ein Testmedium eingebracht, in dem der Analyt enthalten sein kann und dessen Vorhandensein und/oder Konzentration bestimmt wird. Vorteilhaft wird das analytabhängige Sensorsignal, etwa ein konzentrationsabhängiges Sensorsignal, durch den gekoppelten Einsatz des Sensormaterials und des Kompensationsmaterials im Druckmessraum ganz oder wenigstens überwiegend querempfindlichkeitsunabhängig, während die Sensitivität für den Analyten, etwa einen Blutbestandteil, und somit die konzentrationsabhängige Druckänderung jedoch gleichzeitig unverändert bleibt.

Zur Kompensation der Querempfindlichkeitsabhängigkeit des Sensorsignals entkoppelt das Kompensationsmaterial im Biosensor den Querempfindlichkeitseffekt des Sensormaterials mechanisch vom konzentrationsabhängigen Sensorsignal, so dass das konzentrationsabhängige Sensorsignal unabhängig von der Querempfindlichkeit wird.

Günstigerweise kann die querempfindlichkeitsbedingte Volumenänderung des Sensormaterials eine temperaturbedingte Volumenänderung und/oder eine vom pH-Wert bedingte Volumenänderung sein.

Die Erfindung löst ein zentrales Problem von Biosensoren für den Nachweis bzw. die Konzentrationsbestimmung von Substanzen, wobei die Biosensoren vorzugsweise auf einer Quelldruckmessung temperatursensitiver und/oder für den pH-Wert sensitiver Hydrogele basieren. Die Temperaturabhängigkeit und/oder Abhängigkeit vom pH-Wert wird mechanisch von der Sensitivität für den oder die Analyten entkoppelt, so dass ein konzentrationsabhängiger Quelldruck eine verringerte oder gar keine Temperaturabhängigkeit und/oder Abhängigkeit vom pH-Wert mehr aufweist.

Zur Kompensation der Temperaturabhängigkeit und/oder Abhängigkeit vom pH-Wert des Sensorsignals entkoppelt das Kompensationsmaterial im Biosensor den Temperatureffekt und/oder Effekt durch den pH-Wert des Sensormaterials, insbesondere des Hydrogels, mechanisch vom konzentrationsabhängigen Sensorsignal, so dass das konzentrationsabhängige Sensorsignal temperaturunabhängig und/oder unabhängig vom pH-Wert wird.

Günstigerweise kann die Kompensation oder zumindest Verringerung der temperaturbedingten und/oder die durch den pH-Wert bedingte Druckänderung des Hydrogels durch einen mechanischen Aufbau erfolgen. Als Folge können Drucksensoren mit wesentlich kleinerem Messbereich und größerer Messauflösung eingesetzt werden. Damit erhöht sich auch die Messauflösung zur Bestimmung der Sensormessgröße und ermöglicht z.B. die Bestimmung physiologisch relevanter Konzentrationsschwankungen im Implantatsmaßstab.

Vorteilhaft können zur Realisierung neuartiger Biosensoren für die Konzentrationsbestimmung oder den Nachweis von Substanzen sogenannte "smarte" Hydrogele verwendet werden. Smarte Hydrogele sind ein Spezialfall der Hydrogele. Sie reagieren im isobaren Fall (freie Quellung bei Atmosphärendruck) mit Volumenänderungen auf sehr geringe Änderungen ganz bestimmter Umgebungsgrößen (Temperatur, pH-Wert, Ionen- oder Stoffkonzentrationen). Bei solchen Hydrogelen handelt es sich um Polymernetzwerke, welche auf einen Stimulus - hier Änderung der Konzentration einer Substanz in dem getesteten Medium, z.B. Blut - mit einer starken Volumenänderung durch Wasseraufnahme und Wasserabgabe reagieren. Eingebracht in ein definiertes Volumen kann die konzentrationsabhängige Volumenänderung in eine konzentrationsabhängige Druckänderung übersetzt werden. Dies ist beispielsweise bei implantierbaren Biosensoren günstig, mittels derer man die Konzentrationen bestimmter Substanzen, etwa sogenannte Biomarker in Körperflüssigkeiten, wie z.B. Blut, ermitteln kann. Bei den bekannten Systemen sprechen die erforderlichen hohen Anforderungen an die im Biosensor enthaltenen Druck- und Temperatursensoren und/oder Sonden zur Bestimmung eines pH-Werts technisch und ökonomisch klar gegen eine Realisierung von Sensoren auf der Basis von Hydrogelen insbesondere im mobilen Einsatz (z.B. als Implantat). Gemäß der Erfindung ist es nicht notwendig, die Temperatur und/oder den pH-Wert im Messbereich konstant zu halten oder hochpräzise Druck- und Temperatursensoren und/oder Sonden zur Bestimmung eines pH-Werts einzusetzen. Der erfindungsgemäße Biosensor kann dagegen auch in einem Organismus eingesetzt werden, der schwankende Temperaturen und/oder pH-Werte aufweist, etwa bei Fieber oder anderen Einflüssen.

Mit dem erfindungsgemäßen Biosensor kann eine Vielzahl von Substanzen spezifisch nachgewiesen bzw. deren Konzentration bestimmt werden. Hierzu zählen Elektrolyte, Kohlenhydrate, Fettsäuren, Lipide, Zucker, Nucleotide, Desoxyribonukleinsäuren, Ribonukleinsäuren, Aminosäuren, Peptide, Proteine, Antikörper, Hormone, Neurotransmitter, Metaboliten, Stoffwechselprodukte, Antigene, Wirkstoffe, Medikamente, Nanopartikel, Toxine und/oder jeder andere, dem Fachmann für zweckdienlich erscheinende Stoff. Die Sensitivität für die nachzuweisende Substanz, auch Analyt genannt, wird dabei über Komponenten erreicht, die in das Hydrogelnetzwerk z.B. kovalent eingebracht werden.

Nach einer günstigen Ausgestaltung kann ein Volumeneffekt des Sensormaterials bezogen auf eine Querempfindlichkeit des Sensormaterials und auf eine Konzentrationsänderung des Analyten auf das Sensormaterial additiv sein.

Nach einer günstigen Ausgestaltung kann ein Volumeneffekt des Sensormaterials bezogen zum einen auf eine Änderung des pH-Werts und/oder eine Temperaturänderung und zum anderen auf eine Konzentrationsänderung des Analyten auf das Sensormaterial additiv sein.

Für die Erzeugung der "smarten" Hydrogele werden häufig Basismaterialien eingesetzt, die neben der Sensitivität für die jeweiligen Analyten zusätzlich eine starke Temperaturabhängigkeit und/oder Abhängigkeit vom pH-Wert zeigen, d.h. diese Hydrogele können ihren Wassergehalt in Abhängigkeit der Temperatur ändern.

Kann dabei das Hydrogel frei aufquellen oder entquellen, ist dies an einer starken Volumenänderung zu erkennen; ist das Hydrogel auf ein gegebenes Volumen beschränkt, ändert sich dementsprechend der Druck im Volumen. Durch die ausgeprägte Temperaturabhängigkeit und/oder Abhängigkeit vom pH-Wert wird die Wasserabgabe und somit Volumen- bzw. Druckänderung, bei einer Konzentrationsänderung der Substanzen bei konstanter Messtemperatur und/oder bei konstantem pH-Wert verstärkt.

Die Volumen- bzw. Druckänderung des Hydrogels sind somit zum einen abhängig von der Konzentration des Analyten, der die eigentliche Sensormessgröße des Biosensors darstellt und zum anderen der Temperatur und/oder dem pH-Wert. Durch das Kompensationsmaterial kann vorteilhaft das Vorhandensein und/oder die Konzentration des Analyten präzise bestimmt werden, obwohl die Volumen- bzw. Quelldruckänderung bei Variationen der Temperatur und/oder des pH-Werts deutlich größer ist als durch die Änderung der eigentlichen Sensormessgröße, beispielsweise der Konzentration des Blutbestandteils.

Vorteilhaft für die Konzentrationsbestimmung oder den Nachweis medizinisch-, biochemisch-, molekularbiologisch-, biotechnologisch oder umwelttechnisch relevanter Substanzen ist ein Hydrogel. Damit kann der erfindungsgemäße Biosensor zum Nachweis oder zur Konzentrationsbestimmung physiologisch-relevanter Marker, wie z.B. Elektrolyte, Kohlenhydrate und Proteine, eingesetzt werden.

Ein günstiges Hydrogel weist neben der Sensitivität für die Substanz ebenfalls eine Temperaturabhängigkeit und/oder eine Abhängigkeit vom pH-Wert auf, d.h. in Abhängigkeit von der Konzentration der Substanz, der Temperatur und des pH-Werts gibt das Hydrogel Wasser ab, bzw. nimmt es auf, was sich in einem konzentrations- und temperaturabhängigen und vom pH-Wert abhängigen Quelldruck bemerkbar macht, der mit dem Drucksensor bestimmt wird. Die durch Temperatur- und Konzentrationsänderungen und Änderungen des pH-Werts erzeugten Druckänderungen sind dabei additiv.

Ein günstiges Hydrogel weist z.B. einen negativen Zusammenhang zwischen Temperatur und Volumen auf, d.h. bei Temperaturerhöhungen nimmt das Volumen durch Wasserabgabe ab und damit sinkt der gemessene durch Temperaturänderung beeinflusste Druck und bei Temperaturerniedrigungen nimmt das Volumen durch Wasseraufnahme zu und damit steigt der gemessene, durch Temperaturänderung beeinflusste Druck. Um diese Temperaturabhängigkeit teilweise oder vollständig mechanisch zu kompensieren, wird in den Biosensor im Kontakt zum Hydrogel das Kompensationsmaterial eingebracht, welches, im Gegensatz zum Hydrogel, einen entgegengesetzten, nämlich positiven Zusammenhang zwischen Volumen und Temperatur aufweist, d.h. bei Temperaturerhöhungen nimmt das Volumen und damit der gemessene durch Temperaturänderung beeinflusste Druck zu und bei Temperaturerniedrigungen nimmt das Volumen und damit der gemessene, durch Temperaturänderung beeinflusste Druck ab. Hierbei kann der Absolutbetrag der Volumenänderung des Kompensationsmaterials identisch zum Absolutbetrag der Volumenänderung des Hydrogels sein. Ist keine vollständige Kompensation des Temperatureffekts erwünscht, können sich die Absolutbeträge auch unterscheiden.

Für die Ausbildung eines negativen Zusammenhangs zwischen Temperatur und Volumen müssen die Hydrogele eine sogenannte Lower Critical Solution Temperature (untere kritische Lösungstemperatur, LCST) aufweisen. Unterhalb der LCST liegt das Hydrogel gequollen vor (hoher Quelldruck), oberhalb der LCST ist das Hydrogel kollabiert (niedriger Quelldruck). Hierfür sind unter anderem folgende Hydrogele mit LCST denkbar: Poly(N-isopropylacrylamid), Methacrylat-Makromonomere (Ether-Ester-Struktur), Poly(N,N-diethylacrylamid), Poly(N-vinlycaprolactam), Poly[2-(dimethylamino)ethyl methacrylate], Poly(ethylene glycol) (PEG), PEG-methacrylat, Diethylenglykolmethylethermethacrylat, Oligo(ethylenglykol)methylethermethacrylat, Poly(melhylvinylether).

Alternative Hydrogele mit einer "Upper Critical Solution Temperature" (UCST, mit Volumenzunahme bei steigender Temperatur), oder eine Mischung von sowohl UCST und LCST Hydrogelen sind in diesem Zusammenhang ebenso denkbar. Weiterhin sind Hydrogele die sowohl eine UCST als auch eine LCST aufweisen für den Einsatz denkbar.

Ein günstiges Hydrogel weist neben der Temperaturabhängigkeit oder alternativ zur Temperaturabhängigkeit z.B. einen negativen Zusammenhang zwischen pH-Wert und Volumen auf, d.h. bei Erhöhungen des pH-Werts nimmt das Volumen durch Wasserabgabe ab und damit sinkt der gemessene durch Änderung des pH-Werts beeinflusste Druck und bei Erniedrigung des pH-Werts nimmt das Volumen durch Wasseraufnahme zu und damit steigt der gemessene, durch Änderung des pH-Werts beeinflusste Druck. Um diese Abhängigkeit vom pH-Wert teilweise oder vollständig mechanisch zu kompensieren, wird in den Biosensor im Kontakt zum Hydrogel das Kompensationsmaterial eingebracht, welches, im Gegensatz zum Hydrogel, einen entgegengesetzten, nämlich positiven Zusammenhang zwischen Volumen und pH-Wert aufweist, d.h. bei Erhöhung des pH-Werts nimmt das Volumen und damit der gemessene durch Änderung des pH-Werts beeinflusste Druck zu und bei Erniedrigung des pH-Werts nimmt das Volumen und damit der gemessene, durch Änderung des pH-Werts beeinflusste Druck ab. Hierbei kann der Absolutbetrag der Volumenänderung des Kompensationsmaterials identisch zum Absolutbetrag der Volumenänderung des Hydrogels sein. Ist keine vollständige Kompensation des pH-Effekts erwünscht, können sich die Absolutbeträge auch unterscheiden.

Ein günstiges Hydrogel weist für die Ausbildung eines negativen Zusammenhangs zwischen pH-Wert und Volumen basische Gruppen auf, d.h. bei niedrigeren pH-Werten weisen solche Hydrogele ein erhöhtes Volumen auf, während bei höheren pH-Werten das Volumen kleiner ist.

Ein günstiges Hydrogel weist für die Ausbildung eines positiven Zusammenhangs zwischen pH-Wert und Volumen sauere Gruppen auf, d.h. bei höheren pH-Werten weisen solche Hydrogele ein erhöhtes Volumen auf, während bei niedrigeren pH-Werten das Volumen kleiner ist.

Ein günstiges Hydrogel weist für die Ausbildung eines alternierenden Zusammenhangs zwischen pH-Wert und Volumen sowohl basische wie auch saure Gruppen auf, d.h. bei niedrigeren pH-Werten weisen solche Hydrogele ein erhöhtes Volumen auf, bei mittleren pH-Werten ist das Volumen erniedrigt, während bei höheren pH-Werten das Volumen erhöht ist.

Für die Kompensation der jeweiligen Volumenänderung in Abhängigkeit vom pH-Wert können diese Hydrogele zweckdienlich in Sensoren kombiniert werden.

Gemäß einer günstigen Ausgestaltung kann der Druckmessraum wenigstens zwei Kammern aufweisen und das Sensormaterial in einer ersten der beiden Kammern und das Kompensationsmaterial in einer zweiten der beiden Kammern angeordnet sein. Die erste der beiden Kammern kann weiterhin einen Bereich aufweisen, der für den Analyten und das Testmedium durchlässig ist. Die Kammern können unmittelbar aneinandergrenzen oder voneinander beabstandet sein. Im letzteren Fall können die beiden Kammern jedoch miteinander verbunden sein.

Gemäß einer günstigen Ausgestaltung kann zwischen den beiden Kammern eine flexible Membran angeordnet sein. Vorteilhaft können beide Kammern einen Bereich aufweisen, der für den Analyten und das Testmedium durchlässig ist. Kommt das Kompensationsmaterial mit dem den Analyten enthaltenen Testmedium in Kontakt, kann das Kompensationsmaterial auch genutzt werden, um neben der Temperaturabhängigkeit auch andere Querempfindlichkeiten des Sensormaterials zu kompensieren.

Grundsätzlich kann die Erfindung auf alle polymerbasierten Sensoren zur Kompensation von Querempfindlichkeiten erweitert werden. Die Sensitivität des Sensormaterials und des Kompensationsmaterials des erfindungsgemäßen Biosensors auf bestimmte Analyten, z.B. Blutbestandteile kann bei polymerbasierten Systemen wie Hydrogel durch das verwendete Polymersystem bestimmt werden.

Gemäß einer günstigen Ausgestaltung kann der Drucksensor zumindest teilweise in der zweiten Kammer angeordnet und zumindest bereichsweise vom Kompensationsmaterial umgeben sein. Insbesondere kann das Kompensationsmaterial Öl sein. Vorteilhaft kann das Kompensationsmaterial in diesem Fall zur Druckübertragung zwischen dem Sensormaterial in der ersten Kammer und dem Drucksensor in der zweiten Kammer eingesetzt sein. Zweckmäßigerweise ist der Drucksensor vom Öl umgeben. Die zweite Kammer ist in diesem Fall nicht für das Testmedium bzw. den Analyten durchlässig.

Öl weist einen positiven Zusammenhang zwischen Temperatur und Volumen auf, während ein günstiges Hydrogel einen negativen Zusammenhang zwischen Temperatur und Volumen aufweist. Die thermisch bedingte Ausdehnung des verwendeten Öls bei steigender Temperatur und somit der temperaturbedingte Druckanstieg kann mechanisch kompensiert werden.

Alternativ kann das Sensormaterial im Druckmessraum mit dem Kompensationsmaterial gemischt sein, so dass Sensormaterial und Kompensationsmaterial in einer Kammer angeordnet sein können. Diese Kammer kann weiterhin einen Bereich aufweisen, der für den Analyten und das Testmedium durchlässig ist. Sensormaterial und Kompensationsmaterial können ihre Volumenänderungen je nach Materialwahl ganz oder zumindest teilweise kompensieren. Vorteilhaft kann das Sensormaterial mit dem Kompensationsmaterial eine Stapelanordnung mit alternierenden Lagen von Sensormaterial und Kompensationsmaterial bilden. Der Stapel kann durch aufeinander gestapelte Scheiben gebildet sein oder mit einem Beschichtungsauftrag, etwa Spincoating, gebildet sein. Es kann stattdessen auch eine Mischung von Partikeln aus Sensormaterial und Kompensationsmaterial vorgesehen sein.

Gemäß einer günstigen Ausgestaltung kann der Drucksensor innerhalb des Druckmessraums in einer zusätzlichen Kammer angeordnet sein. Diese Kammer kannabhängig von der Art des Drucksensors - mit einem Druckübertragungsmedium gefüllt sein. Die Anordnung des Drucksensors innerhalb des Druckmessraums in einer zusätzlichen Kammer ist dann zweckmäßig, wenn das Sensormaterial und das Kompensationsmaterial in einer Kammer gemischt vorliegt oder in zwei benachbarten Kammern angeordnet ist und das Kompensationsmaterial nicht das Übertragungsmedium für den Druck ist, in dem der Drucksensor angeordnet ist.

Gemäß einer günstigen Ausgestaltung kann der Drucksensor außerhalb des Druckmessraums in einer zusätzlichen Kammer angeordnet sein, die mit einem Druckübertragungsmedium gefüllt ist. Dies ist dann zweckmäßig, wenn das Sensormaterial und das Kompensationsmaterial in einer Kammer gemischt vorliegen oder in zwei benachbarten Kammern angeordnet sind und das Kompensationsmaterial nicht das Übertragungsmedium für den Druck ist, in dem der Drucksensor angeordnet ist.

Gemäß einer günstigen Ausgestaltung kann der Drucksensor mit der Wandung des Druckmessraums verbunden sein oder ein oder mehrere Bestandteile des Drucksensors können ein Bestandteil der Wandung des Druckmessraums sein. Eine derartige Anordnung des Drucksensors kann dann zweckmäßig sein, wenn mechanische Verformungen des Druckmessraums für die Druckmessung zum Einsatz kommen.

Gemäß einer günstigen Ausgestaltung kann die erste Kammer mit der zweiten Kammer durch eine Druckkolbenverbindung so gekoppelt sein, dass eine Volumenänderung der ersten Kammer zu einer Volumenänderung der zweiten Kammer führt.

Gemäß einer günstigen alternativen Ausgestaltung kann die erste Kammer mit der zweiten Kammer durch eine dehnbare Rohrverbindung so gekoppelt sein, dass eine Volumenänderung der ersten Kammer zu einer Volumenänderung der zweiten Kammer führt. Dies erlaubt eine günstige Abstimmung von Abmessungen des Biosensors und des Druckmessraums.

Gemäß einer günstigen Ausgestaltung kann das Kompensationsmaterial ein Hydrogel sein. Das Hydrogel weist einen dem Sensormaterial entgegengesetzten Zusammenhang von Temperatur und/oder pH-Wert und Volumen auf, wobei der Absolutbetrag der Volumenänderung des Kompensationsmaterials identisch zum Absolutbetrag der Volumenänderung des Sensormaterials sein kann. Ist keine vollständige Kompensation des Temperatureffekts und/oder des pH-Werteffekts erwünscht, können sich die Absolutbeträge auch unterscheiden.

Nach einer günstigen Ausgestaltung kann der Biosensor zum Nachweis oder zur Konzentrationsmessung von Bestandteilen im Blut eingesetzt werden, insbesondere zum Nachweis oder zur Konzentrationsmessung von Glukose oder Kalium.

Vorteilhaft erlaubt die Erfindung den spezifischen Nachweis oder die Konzentrationsbestimmung einer Vielzahl von Substanzen durchzuführen bzw. deren Konzentration zu bestimmen. Hierzu zählen Elektrolyte, Kohlehydrate, Fettsäuren, Lipide, Zucker, Nucleotide, Desoxyribonukleinsäuren, Ribonukleinsäuren, Aminosäuren, Peptide, Proteine, Antikörper, Hormone, Neurotransmitter, Metaboliten, Stoffwechselprodukte, Antigene, Wirkstoffe, Medikamente, Nanopartikel, Toxine und/oder jeder andere, dem Fachmann für zweckdienlich erscheinende Stoff. Die Sensitivität für die nachzuweisende Substanz (Analyt) wird dabei über Molekülteile erreicht, die in das Hydrogelnetzwerk z.B. kovalent eingebracht werden

Die Erfindung ist nachfolgend beispielhaft, anhand von in Zeichnungen dargestellten Ausführungsbeispielen, näher erläutert. Es zeigen in schematischer Darstellung:
- Fig. 1: in Schnittdarstellung eine erste Ausgestaltung eines Biosensors nach einem ersten Ausführungsbeispiel der Erfindung;
- Fig. 2: in Schnittdarstellung eine erste Ausgestaltung eines Biosensors nach einem weiteren Ausführungsbeispiel der Erfindung;
- Fig. 3: in Schnittdarstellung eine erste Ausgestaltung eines Biosensors nach einem weiteren Ausführungsbeispiel der Erfindung;
- Fig. 4: in Schnittdarstellung eine erste Ausgestaltung eines Biosensors nach einem weiteren Ausführungsbeispiel der Erfindung;
- Fig. 5: in Schnittdarstellung eine erste Ausgestaltung eines Biosensors nach einem weiteren Ausführungsbeispiel der Erfindung; und
- Fig. 6: ein Schaubild mit reversibler Verschiebung der Druckänderung durch Bindung von Kalium durch ein kaliumsensitives Hydrogel.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

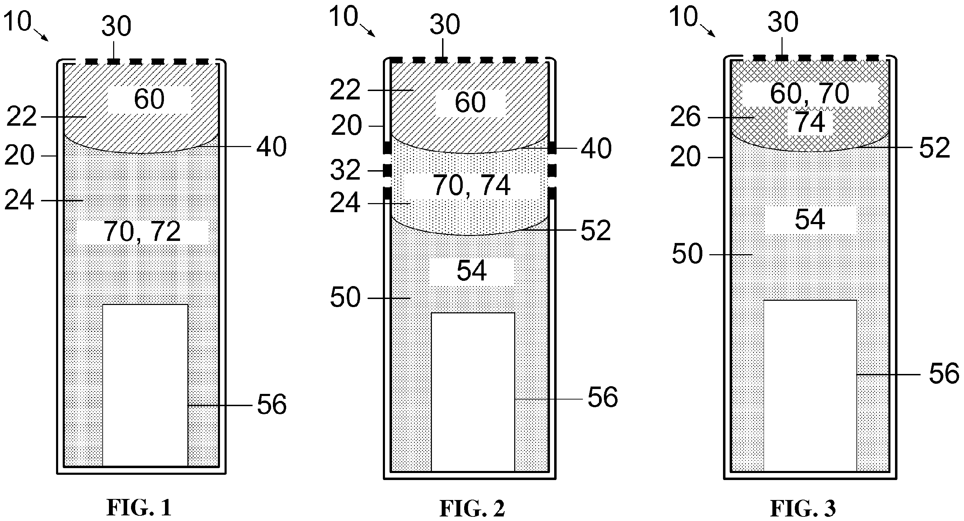

Figur 1 zeigt zur Erläuterung der Erfindung ein erstes Ausführungsbeispiel eines Biosensors 10 nach der Erfindung. Der Biosensor 10 kann insbesondere ein implantierbarer Biosensor sein und weist ein für wenigstens einen Analyten sensibles Sensormaterial 60, insbesondere mit einem für wenigstens einen Analyten sensibles Hydrogel, auf Das Sensormaterial 60 ist in einer Kammer 22 eines Druckmessraums 20 angeordnet und mit einem Kompensationsmaterial 70 in einer zweiten Kammer 24 des Druckmessraums 20 gekoppelt. Im gezeigten Ausführungsbeispiel bildet Öl 72 das Kompensationsmaterial 70.

Die beiden Kammern 22, 24 sind durch eine flexible Membran 40 getrennt. Die Membran 40 ist flexibel, damit sie eine Volumenänderung des Sensormaterials 60 an das Kompensationsmaterial 70 weitergeben kann. Das Kompensationsmaterial 70 weist einen zum Sensormaterial 60 entgegengesetzten Zusammenhang zwischen Temperatur und Volumen auf, so dass eine durch eine Temperaturänderung bewirkte Volumenänderung des Sensormaterials 60 und des Kompensationsmaterials 70, wenn beide Materialien 60, 70 vollständig aufeinander abgestimmt sind, vollständig kompensiert wird.

In grober Näherung ist das Gesamtvolumen des Hydrogels (Sensormaterial 60) durch dessen Verkapselung in der ersten Kammer 22 des Druckmessraums 20 konstant (isochores Verhalten). Der Druck steigt, wenn der Quelldruck des Hydrogels (Sensormaterial 60) in der Kammer 22 zunimmt. Er ist abhängig von der Querempfindlichkeit (z.B. Temperatur) und dem Messeffekt, z.B. einer Änderung der Konzentration des Analyten im Testmedium.

Ein Drucksensor 56, z.B. ein ASIC-Baustein, befindet sich in Öl 72 in der zweiten Kammer 24. Hier wird der Druck des umgebenden Öls 72 gemessen. Der Messdruck wird über die Membran 40 an das Öl 72 und den darin eingebetteten Drucksensor 56 geleitet. Über der Membran 40 ist das Hydrogel als Sensormaterial 60 angekoppelt. Das Testmedium steht über ein für das Medium und den Analyten durchlässiges mechanisches Widerlager 30, das in Form einer porösen Membran ausgeführt sein kann, mit dem Hydrogel (Sensormaterial 60) in Kontakt.

Das Sensormaterial 60 stellt einen chemomechanischen Wandler dar, welcher die Konzentration des Testmediums in einen physikalischen Druck wandelt. Der Drucksensor 56 entspricht einem mechanoelektrischen Wandler, welcher den physikalischen Druck in ein weiterverarbeitbares Signal umformt, aus dem z.B. eine Analytkonzentration im Testmedium bestimmt werden kann.

Als Sensormaterial 60 sind insbesondere "smarte" Hydrogele geeignet. Smarte Hydrogele sind ein Spezialfall der Hydrogele. Sie reagieren im isobaren Fall (freie Quellung bei Atmosphärendruck) mit Volumenänderungen auf sehr geringe Änderungen ganz bestimmter Umgebungsgrößen (Temperatur, pH-Wert, Ionen- oder Stoffkonzentrationen). Durch die entstehenden Quellungskräfte können die smarten Hydrogele mechanische Arbeit verrichten. Im isochoren Fall, d.h. bei konstantem Volumen, kann die Änderung der Umgebungsgröße zu einer Druckänderung führen. Die nachzuweisenden Analyten können dabei durch das für das Medium und den Analyten durchlässige mechanische Widerlager 30, das in Form einer porösen Membran ausgeführt sein kann, von außen in das Hydrogel eindringen. Durch die Kombination "Reaktion auf Umgebungsgröße" und "Änderung des Volumens oder des Drucks" weisen Hydrogele sowohl Sensorfunktionalitäten als auch Aktorfunktionalitäten auf.

Diese Ausgestaltung ist für LCST-Hydrogele als Sensormaterial 60 geeignet, die einen negativen Zusammenhang zwischen Temperatur und Volumen aufweisen.

Der Grad der Kompensation des Temperatureffekts von Sensormaterial 60 und Kompensationsmaterial 70 erfolgt durch Abstimmung von Hydrogelvolumen und Ölvolumen in den beiden Kammern 22, 24.

LCST-Hydrogele quellen mit sinkender Temperatur, so dass der Quelldruck zunimmt (Querempfindlichkeit). Die durch den Messeffekt verursachte Quelldruckänderung (z.B. durch Konzentrationsänderung im Testmedium) ist deutlich kleiner als der Temperatureffekt. Das Kompensationsmaterial 70 in Form von Öl 72 zieht sich mit sinkender Temperatur zusammen. Die Querempfindlichkeit kann kompensiert werden, indem das temperaturbedingte Quellen des Hydrogels (Sensormaterial 60) durch die Volumenabnahme des Öls 72 ausgeglichen wird.

Die Sensitivität für die Umgebungsgrößen wird dabei durch Einsatz von Hydrogelbausteinen erreicht, welche spezielle Affinitäten für externe Stimuli besitzen. Beispielsweise können diese Bausteine (und hierdurch auch die Hydrogele) Stoffe spezifisch z.B. nach dem Schlüssel-Schloss-Prinzip, Komplexierung oder anderen physikochemischen Wechselwirkungen erkennen.

Um eine vollständige Kompensation des Temperatureffekts in Sensormaterial 60 und im Kompensationsmaterial zu erhalten (1:1 Übersetzung), ergibt sich folgende Abschätzung des Verhältnisses Ölvolumen / Hydrogeltrockenmasse. Kompensation bedeutet, dass ΔV_{Öl}/ΔT= -ΔV_{HG}/ΔT, d.h. die temperaturbedingte Änderung des Ölvolumens entspricht der temperaturbedingten Änderung des Hydrogelvolumens begleitet durch Wasseraufhahme/-abgabe.

Für ein vorliegendes Hydrogel (Sensormaterial 60) ergibt sich als Abschätzung ein Verhältnis V₇₂/m₆₀ des Ölvolumens V₇₂ zur Trockenmasse des Hydrogels m₆₀ (Sensormaterial 60) V₇₂/m₆₀=360 µl/mg. Vorteilhaft in einem implantierbaren Biosensor 10 ist eine Befüllung mit m₆₀=2 mg. Demnach wäre zur vollständigen Kompensation ein Ölvolumen V₇₂ von 720 µl nötig. Das Hydrogel (Sensormaterial 60) kann bzgl. der Quelleigenschaften angepasst werden, wodurch die Temperaturabhängigkeit des Quellens reduziert und erhöht werden kann. Somit kann hierdurch die erforderliche Ölmenge V₇₂ reduziert oder erhöht werden. Es sind somit auch Hydrogele als Sensormaterial 60 denkbar, bei denen das Verhältnis drastisch anders ist, beispielsweise auch 10.000 µl/mg, 1.000 µl/mg, 100 µl/mg, 10 µl/mg, 1 µl/mg, 0,1 µl/mg. Wird keine vollständige Kompensation oder eine Überkompensation der Temperaturabhängigkeit gewünscht, kann das Verhältnis V₇₂/m₆₀ des Ölvolumens V₇₂ zur Trockenmasse entsprechend angepasst werden.

Geringe Mengen der Hydrogele (Sensormaterial 60) können z.B. über Spincoating in die Kammer 22 des Biosensors 10 eingebracht werden, bei größeren Mengen ist ein Einsatz von Partikeln oder auch Scheiben möglich. Die Hydrogele (Sensormaterial 60) können auch direkt im Biosensor 10 synthetisiert werden.

Die Membran 40 zwischen der Kammer 22 mit dem Sensormaterial 60 und der Kammer 24 mit dem Kompensationsmaterial 70, in diesem Fall Öl 72, muss dabei sehr flexibel sein.

Figur 2 zeigt eine weitere Ausgestaltung eines Biosensors nach der Erfindung. Das Sensormaterial 60 ist vorteilhaft ein Hydrogel wie in Figur 1 beschrieben wurde. Allerdings kann sowohl ein LCST-Hydrogel als auch ein UCST-Hydrogel eingesetzt werden.

Im Gegensatz zum Beispiel in Figur 1 ist der Drucksensor 56 in einer separaten Kammer 50 angeordnet, die an die Kammer 24 angrenzt und durch eine Membran 52 von dieser getrennt ist. Der Drucksensor 56 ist in Öl als Übertragungsmedium 54 eingebettet.

Das Sensormaterial 60 ist wiederum in einer ersten Kammer 22 und das Kompensationsmaterial 70 in einer zweiten Kammer 24 angeordnet. Im Gegensatz zu Figur 1 ist das Kompensationsmaterial 70 kein Öl, sondern ein Hydrogel 74, das eine dem Sensormaterial 60 entgegengesetzten Zusammenhang zwischen Volumen und Temperatur aufweist.

Die erste Kammer 22 mit dem Sensormaterial 60 ist durch ein für das Medium und den Analyten durchlässiges mechanisches Widerlager 30, das in Form einer porösen Membran ausgeführt sein kann, mit einem Testmedium in Kontakt. Ebenso ist das Kompensationsmaterial 70 mit einem zumindest bereichsweise an den Seitenwänden der zweiten Kammer 24 angeordneten, für das Medium und den Analyten durchlässiges mechanisches Widerlager 32, das in Form einer porösen Membran ausgeführt sein kann, mit einem Testmedium in Kontakt. Beide Kammern 22, 24 sind durch eine flexible Membran 40 getrennt, die flexibel ist, um die Volumenkompensation zwischen Kammer 22 und Kammer 24 zu ermöglichen.

Eine Kompensation des Temperatureffekts erfolgt durch Abstimmung von Hydrogel (Sensormaterial 60) und Hydrogel 74 (Kompensationsmaterial 70).

Hydrogele als Sensormaterial 60 mit anderen Querempfindlichkeiten (z.B. pH-Wert) können über das Hydrogel 74 als Kompensationsmaterial ausgeglichen werden. Beide Hydrogele 60 und 74 werden vom Testmedium umspült.
Das Kompensationsmaterial 70 ändert sein Volumen in Abhängigkeit der Querempfindlichkeit des Hydrogels als Sensormaterial 60 mit gleichem Absolutbetrag und umgekehrten Vorzeichen. Der Quelldruck in der Druckmesskammer 20 ist nur noch vom Messeffekt abhängig. Um die Temperaturabhängigkeiten, Abhängigkeiten vom pH-Wert und/oder Abhängigkeiten von anderen Quereffekten, der Hydrogele 60, 74 aneinander anzupassen, können die Hydrogele 60, 74 in weiten Bereichen z.B. über Quervernetzungsgrad, eingesetzte Polymeranteil und dergleichen, bei der Synthese modifiziert werden.

Es kann eine übliche Membran 52 zwischen Kammer 24 und Kammer 50 vorgesehen sein, bei der die Ansprüche an eine Membranflexibilität nicht so hoch sind, da es bei vollständiger Volumenkompensation zwischen Sensormaterial 60 und Kompensationsmaterial 70 zu keiner Membrandeflexion durch Querempfindlichkeit kommt.

Figur 3 zeigt eine weitere Ausgestaltung eines erfindungsgemäßen Biosensors 10. Das Sensormaterial 60 ist vorteilhaft ein Hydrogel und kann wie in Figur 2 beschrieben wurde, ein LCST-Hydrogel oder ein UCST-Hydrogel sein. Ebenso ist das Kompensationsmaterial 70 ein Hydrogel 74.

Das Sensormaterial 60 ist im Druckmessraum 20 in einer Kammer 26 mit dem Kompensationsmaterial 70 gemischt und kann dabei eine Stapelanordnung mit alternierenden Lagen von Sensormaterial 60 und Kompensationsmaterial 70 bilden oder eine Mischung von Partikeln aus Sensormaterial 60 und Kompensationsmaterial 70 sein. Durch das für das Medium und den Analyten durchlässige mechanische Widerlager 30, das in Form einer porösen Membran ausgeführt sein kann, können Sensormaterial 60 und Kompensationsmaterial 70 in der Kammer 26 mit einem Testmedium in Kontakt kommen.

Der Drucksensor 56 ist angrenzend an die Kammer 26 in einer Kammer 50 angeordnet, die mit einem Druckübertragungsmedium 54, z.B. Öl, gefüllt ist. Zwischen Kammer 26 und Kammer 50 ist eine Membran 52 angeordnet.

Eine Kompensation des Temperatureffekts in Sensormaterial 60 und Kompensationsmaterial 70 erfolgt durch Mischung des Hydrogels des Sensormaterials 60 und des Hydrogels des Kompensationsmaterials 70.

Diese Variante hat den Vorteil, dass keine Membran zwischen Sensormaterial 60 und Kompensationsmaterial 70 notwendig ist. Es kann eine übliche Membran 52 zwischen Kammer 26 und Kammer 50 vorgesehen sein, bei der die Ansprüche an eine Membranflexibilität nicht so hoch sind, da es bei vollständiger Volumenkompensation zwischen Sensormaterial 60 und Kompensationsmaterial 70 zu keiner Membrandeflexion durch Querempfindlichkeit kommt.

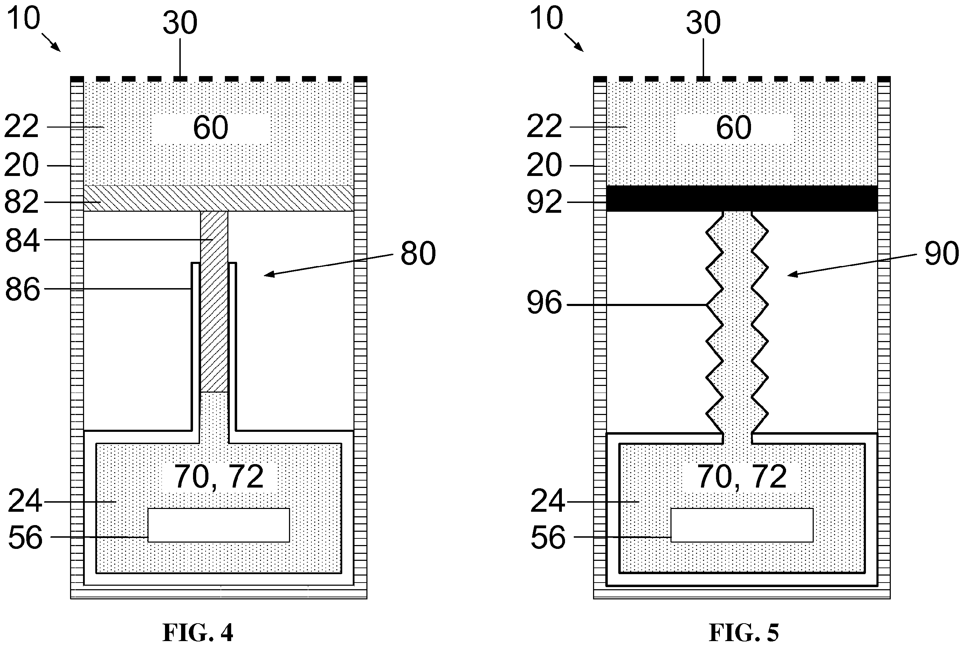

Die Figuren 4 und 5 zeigen zwei weitere Ausführungsbeispiele von Biosensoren 10, bei denen das Kompensationsmaterial 70 wie im Ausführungsbeispiel in Figur 1 ein Öl 72 ist, das einen dem Sensormaterial 60 entgegengesetzten Zusammenhang zwischen Volumen und Temperatur aufweist. Das Sensormaterial 60 ist in einer ersten Kammer 22 und das Kompensationsmaterial 70 in einer zweiten Kammer 24 des Druckmessraums 20 angeordnet. Das Testmedium steht über ein für das Medium und den Analyten durchlässiges mechanisches Widerlager 30, das in Form einer porösen Membran ausgeführt sein kann, mit dem Hydrogel (Sensormaterial 60) in Kontakt.

Ein Drucksensor 56, z.B. ein ASIC-Baustein, befindet sich in Öl 72 in der zweiten Kammer 24. Hier wird der Druck des umgebenen Öls 72 gemessen.

Die Ausgestaltungen erlauben im Vergleich zum Ausführungsbeispiel in Figur 1 eine Reduktion/Erhöhung des pro Trockenmasse Hydrogel (Sensormaterial 60) erforderlichen Ölvolumens durch einen hydraulischen Ansatz.

In Figur 4 wird die Volumenänderung des Sensormaterials 60 in Kammer 22 über eine Druckkolbenverbindung 80 an das Öl 72 in Kammer 24 und den darin eingebetteten Drucksensor 56 geleitet.

Die Verstärkung der Ölausdehnung zur Kompensation des Ölvolumens in der zweiten Kammer 24 wird über die Druckkolbenverbindung 80 erreicht. Dabei wird bei einer Volumenzunahme des Öls 72 ein in einer Führung 86 geführter Kolben 84 nach oben gedrückt. Durch den Hubtisch 82 wird dann das Volumen der Druckmesskammer 22, in dem sich das Hydrogel als Sensormaterial 60 befindet, abhängig zur Temperatur korrigiert.

In Figur 5 wird die Volumenänderung des Sensormaterials 60 in Kammer 22 über eine dehnbare Rohrverbindung 90 an das Öl 72 in Kammer 24 und den darin eingebetteten Drucksensor 56 geleitet.

Die Verstärkung der Kompensation durch die Ölausdehnung in der zweiten Kammer 24 wird über die dehnbare Rohrverbindung 90 erreicht. Dabei wird bei einer Volumenzunahme des Öls 72 der Hubtisch 92 nach oben gedrückt. Durch den Hubtisch 92 wird dann das Volumen der Kammer 22, in dem sich das Hydrogel als Sensormaterial 60 befindet, abhängig zur Temperatur korrigiert.

Für die Abschätzung des Verhältnisses Ölvolumen / Hydrogeltrockenmasse bei 1:n Übersetzung (1:n-Übersetzung bedeutet, dass über ein Verstärkerprinzip die Volumenänderung des Öls 72 zu einem n-fachen Kompensationsvolumen am Hydrogel (Sensormaterial 60) führt).

Eine Verstärkung kann durch Reduktion des Querschnitts der Mechanik, d.h. des Kolbenquerschnitts A84 in Figur 4 oder des Rohrquerschnitts A96 in Figur 5 gegenüber der Fläche des Hubtischs 82 (A₈₂), 92 (A₉₂) entsprechend der Hydrogelfläche erfolgen. Die Querschnittsflächen A₈₄ und A₉₆ werden auch als AM, die Querschnittsflächen A₈₂ und A₉₂ auch als _{AH} bezeichnet.

Kompensation bedeutet, dass die verstärkte temperaturbedingte Änderung des Ölvolumens ΔV₇₂ der temperaturbedingten Änderung des Hydrogelvolumens ΔV₆₀ begleitet durch Wasseraufhahme/-abgabe entspricht, mit n*ΔV₇₂/ΔT= -ΔV₆₀/ΔT.

In der Ausführung als Hubtisch 82, 92 ergibt sich für ein vorliegendes Hydrogel (Sensormaterial 60) ein Verhältnis des Volumens V₇₂ des Öls 72 zur Trockenmasse m₆₀ des Hydrogels (Sensormaterial 60) von V₇₂/m₆₀ von 360 µl/mg. Ohne Übersetzung ist zur vollständigen Kompensation bei einer Befüllung mit m₆₀=2 mg ein Ölvolumen von V₇₂=720 µl nötig. Eine mögliche Übersetzung ergibt sich aus _{AM}= 10 *_{AH}. Mit der beschriebenen Übersetzung n=10 reduziert sich zur vollständigen Kompensation bei einer Befüllung mit m₆₀=2 mg das Ölvolumen V₇₂ auf 72 µl. Eine Anpassung der Hydrogelmenge ist auch bei 1:n Übersetzung möglich. Wird keine vollständige Kompensation oder eine Überkompensation der Temperaturabhängigkeit gewünscht, kann das Verhältnis V₇₂/m₆₀ des Ölvolumens V₇₂ zur Trockenmasse m₆₀ entsprechend angepasst werden.

Vorteilhaft können die vorstehend beschriebenen Ausführungen in den Figuren 1-5 des Biosensors 10 zum Nachweis oder zur Konzentrationsmessung von Bestandteilen im Blut als Testmedium eingesetzt werden. Da der Messeffekt vom Material des verwendeten Hydrogels (Sensormaterial 60) abhängig ist, trägt der Aufbau einen Plattformcharakter. So entsteht beispielsweise bei einem glukosesensitiven Hydrogel als Sensormaterial 60 ein Glukosesensor.

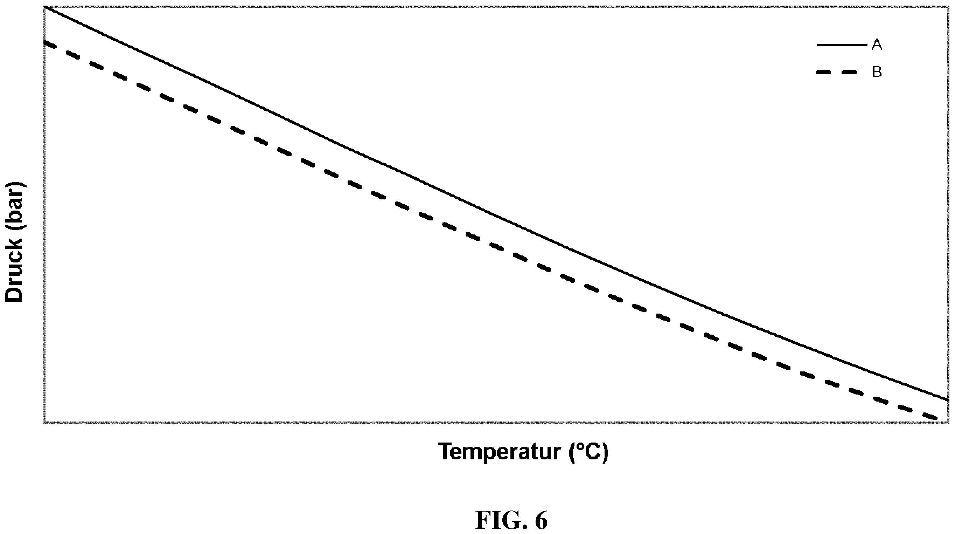

Die Figur 6 zeigt Messkurven A, B eines kaliumsensitiven Hydrogels für einen implantatstypischen Temperaturbereich. Kurve A zeigt einen Verlauf des Drucks p über der Temperatur T ohne Kalium im Testmedium. Kurve B zeigt den Verlauf mit Kaliumzugabe.

Im isochoren Fall, d.h. bei konstantem Volumen des Sensormaterials 60, zeigt das Hydrogel eine kaliumabhängige Verschiebung der Druck-Temperaturkurve. Die Zugabe von KNO₃ verschiebt die Kurve von A nach B nach links in der Figur 6.

Die Temperaturabhängigkeit des Volumens und damit auch des Drucks beeinflusst dabei maßgeblich die Sensitivität. Diese Abhängigkeit ist zum einen vom Hydrogelrezept abhängig. Beispielsweise kann man durch einen geringeren Einsatz der verwendeten Quervernetzer durch die hierdurch reduzierten Rückstellkräfte eine größere temperaturabhängige Volumen- und damit Druckänderung beobachten. Zum anderen kann auch der Füllgrad der Hydrogele (Sensormaterial 60 und Kompensationsmaterial 70) im Druckmessraum 20 des kaliumsensitiven Biosensors 10 die temperaturabhängige Druckänderung beeinflussen. Unter dem Füllgrad versteht man die eingesetzte Trockenmasse des Hydrogels pro Volumen. Bringt man mehr Trockenmasse des Hydrogels (Sensormaterial 60 und Kompensationsmaterial 70) in das vorgegebene Volumen der Druckmesskammer 20 ein, wird der Anstieg der Druck-Temperaturkurven erhöht, was bei der Messtemperatur und einem gegebenen Zusammenhang zwischen Volumen (Druck) und Temperatur eine höhere Sensitivität des Biosensors 10 bewirkt.

Es ist damit möglich, mit Hydrogelen, welche Erkennungskomponenten für Kalium enthalten, sich ändernde Kaliumkonzentrationen über Druckänderungen zu detektieren. Mit dem erfindungsgemäßen Biosensor 10 kann die schwierige Aufgabe gelöst werden, die Temperaturänderungen von Änderungen der Kaliumkonzentration zu trennen. Technische Betrachtungen haben gezeigt, dass eine Bestimmung der Temperatur in dem erforderlichen Maße technisch insbesondere im Implantatsmaßstab, die für eine Messung ohne Temperaturkompensation notwendig wäre, nur schwer zu realisieren ist. Am beispielhaft gezeigten kaliumsensitiven Hydrogel ergibt sich zusammen mit der Forderung nach einer Auflösung von 0,1 mM Kaliumkonzentration eine zur Bewertung des Temperatur- und Kaliumeinflusses am Messdruck notwendige Temperaturauflösung ohne Anwendung des erfindungsgemäßen Sensors von unter 7,5 mK. Bei einer vollständigen Kompensation durch den erfindungsgemäßen Sensor entfällt die Temperaturmessung vollständig. Gelingt die Kompensation unter Anwendung des erfindungsgemäßen Sensors mit einer Toleranz von 5%, so erhöht sich die notwendige Temperaturmessauflösung um den Faktor 20 auf 150 mK. Dies ist ein Wert, der von integrierten Temperatursensoren der Mikrosystemtechnik gut erreicht wird. Gleichzeitig bleibt die Sensitivität für den nachzuweisenden Analyten (ermittelt durch kaliumabhängige Druckänderung) davon unbeeinflusst.

Das Kompensationsmaterial 70 weist für eine vollständige Beseitigung der temperaturabhängigen Druckänderung des Sensormaterials 60 in Form von LCST-Hydrogel einen positiven Zusammenhang zwischen Volumen und Temperatur auf, wobei die temperaturabhängige Volumenänderung des Kompensationsmaterials die temperaturabhängige Volumenänderung des Sensormaterials kompensiert. Hierdurch wird dem Hydrogel bei Erwärmung weniger und bei Abkühlung mehr Volumen zur Verfügung gestellt. Dieser Vorgang erfolgt isobar, d.h. ohne Druckänderung.

Die Kaliumsensitivität bleibt davon unbeeinflusst, weil das Hydrogel (Sensormaterial 60) weiterhin in Abhängigkeit der Kaliumkonzentration Wasser aufnimmt/abgibt. Diese Änderung erfolgt isochor, d.h. bei konstantem Volumen. Durch Entkopplung der beiden Effekte (Temperaturabhängigkeit und Kaliumsensitivität) über Aufteilung in isobare (→ Kompensation der Temperaturabhängigkeit) und isochore (→Kaliumsensitivität) Zustandsänderungen können die Kaliumkonzentrationen bei sich ändernden Temperaturen mit einer hohen Präzision ermittelt werden. Die Kombination von Sensormaterial 60 und Kompensationsmaterial 70 mit gegenläufigen Temperaturabhängigkeiten in Bezug auf Druck und Volumen ermöglicht somit eine Kombination isochorer und isobarer Zustandsänderungen. Eine hochgenaue, technisch insbesondere im Implantatsmaßstab schwer zu realisierende Temperaturmessung ist in solchen Fällen nicht mehr erforderlich.

Das Kompensationsmaterial 70 kann auch so ausgebildet sein, dass andere Querempfindlichkeiten (z.B. pH-Wert) des eingesetzten Hydrogels, bzw. aller einsetzbaren Polymere und Testmedien) kompensiert werden können.

## Patentansprüche

1. Biosensor (10) mit einem für wenigstens einen Analyten sensiblen Sensormaterial (60) in einem mit einem Drucksensor (56) gekoppelten Druckmessraum (20), wobei ein im Druckmessraum (20) herrschender Druck durch den Drucksensor (56) bestimmbar ist,
**dadurch gekennzeichnet, dass** das Sensormaterial (60) im Druckmessraum (20) mit einem Kompensationsmaterial (70) gekoppelt ist, welches eine querempfindlichkeitsbedingte Volumenänderung des Sensormaterials (60) zumindest teilweise kompensiert.

2. Biosensor nach Anspruch 1, **dadurch gekennzeichnet, dass** die querempfindlichkeitsbedingte Volumenänderung des Sensormaterials (60) eine temperaturbedingte Volumenänderung und/oder eine vom pH-Wert bedingte Volumenänderung ist.

3. Biosensor nach Anspruch 1 oder 2, **dadurch gekennzeichnet, dass** es sich bei dem für wenigstens einen Analyten sensiblen Sensormaterial (60) um ein für wenigstens einen Analyten sensibles Polymergel, oder Hydrogel oder "smartes" Hydrogel handelt.

4. Biosensor nach einem der Ansprüche 1-3, **dadurch gekennzeichnet, dass** ein Volumeneffekt des Sensormaterials (60) bezogen zum einen auf eine Änderung des pH-Werts und/oder eine Temperaturänderung und zum anderen auf eine Konzentrationsänderung des Analyten auf das Sensormaterial (60) additiv ist.

5. Biosensor nach einem der Ansprüche 1-4, **dadurch gekennzeichnet, dass** der Druckmessraum (20) wenigstens zwei Kammern (22, 24) aufweist und das Sensormaterial (60) in einer ersten der beiden Kammern (22) und das Kompensationsmaterial (70) in der zweiten der beiden Kammern (24) angeordnet ist, wobei die erste Kammer (22) weiterhin einen Bereich (30) aufweist, der für den Analyten und das Testmedium durchlässig ist.

6. Biosensor nach Anspruch 5, **dadurch gekennzeichnet, dass** die Kammern (22; 24) aneinander grenzen und/oder dass zwischen den beiden Kammern (22; 24) eine flexible Membran (40) angeordnet ist.

7. Biosensor nach Anspruch 6, **dadurch gekennzeichnet, dass** beide Kammern (22, 24) einen Bereich (30, 32) aufweisen, der für den Analyten und das Testmedium durchlässig ist.

8. Biosensor nach einem der Ansprüche 5 oder 6, **dadurch gekennzeichnet, dass** der Drucksensor (56) zumindest teilweise in der zweiten Kammer (24) angeordnet und zumindest bereichsweise vom Kompensationsmaterial (70) umgeben ist.

9. Biosensor nach Anspruch 8, **dadurch gekennzeichnet, dass** das Kompensationsmaterial (70) Öl (72) ist.

10. Biosensor nach einem der Ansprüche 1-4, **dadurch gekennzeichnet, dass** das Sensormaterial (60) im Druckmessraum (20) mit dem Kompensationsmaterial (70) gemischt ist, so dass das Sensormaterial (60) und das Kompensationsmaterial (70) in einer Kammer (26) angeordnet ist und diese Kammer weiterhin einen Bereich (30) aufweist der für den Analyten und das Testmedium durchlässig ist.

11. Biosensor nach Anspruch 10, **dadurch gekennzeichnet, dass** das Sensormaterial (60) mit dem Kompensationsmaterial (70) eine Stapelanordnung mit alternierenden Lagen von Sensormaterial (60) und Kompensationsmaterial (70) bildet oder dass eine Mischung von Partikeln aus Sensormaterial (60) und Kompensationsmaterial (70) vorgesehen ist.

12. Biosensor nach einem der Ansprüche 1-7 oder 10-11, **dadurch gekennzeichnet, dass** der Drucksensor (56) innerhalb des Druckmessraums (20) in einer zusätzlichen Kammer (50) angeordnet ist, die mit einem Druckübertragungsmedium (54) gefüllt sein kann.

13. Biosensor nach einem der Ansprüche 1-5, **dadurch gekennzeichnet, dass** die erste Kammer (22) mit der zweiten Kammer (24) durch eine Druckkolbenverbindung (80) oder durch eine dehnbare Rohrverbindung (90) so gekoppelt ist, dass eine Volumenänderung der ersten Kammer (22) zu einer Volumenänderung der zweiten Kammer (24) führt.

14. Biosensor nach einem der Ansprüche 1-7 oder 10-11, **dadurch gekennzeichnet, dass** das Kompensationsmaterial (74) ein Hydrogel ist.

15. Biosensor nach einem der vorhergehenden Ansprüche, **dadurch gekennzeichnet, dass** der Biosensor (10) in den menschlichen oder tierischen Körper implantierbar ist.

16. Biosensor nach einem der Ansprüche 1-7, 10-11 oder 13-14, **dadurch gekennzeichnet, dass** der Drucksensor (56) mit der Wandung des Druckmessraums (20) verbunden ist oder ein oder mehrere Bestandteile des Drucksensors (56) ein Bestandteil der Wandung des Druckmessraums (20) ist.

## Claims

1. A biosensor (10), comprising sensor material (60) sensitive for at least one analyte in a pressure measurement chamber (20) coupled to a pressure sensor (56), wherein a pressure prevailing in the pressure measurement chamber (20) can be determined by the pressure sensor (56),
**characterised in that** the sensor material (60) in the pressure measurement chamber (20) is coupled to a compensation material (70), which at least partially compensates for a cross-sensitivity-induced change in volume of the sensor material (60).

2. The biosensor according to claim 1, **characterised in that** the cross-sensitivity-induced change in volume of the sensor material (60) is a temperature-induced change in volume and/or a pH value-induced change in volume.

3. The biosensor according to claim 1 or 2, **characterised in that** the sensor material (60) sensitive to at least one analyte is a polymer gel, or hydrogel, or a smart hydrogel sensitive to at least one analyte.

4. The biosensor according to any one of claims 1-3, **characterised in that** a volume effect of the sensor material (60) based on the one hand on a change to the pH value and/or a temperature change and based on the other hand on a change in concentration of the analyte is additive at the sensor material (60).

5. The biosensor according to any one of claims 1-4, **characterised in that** the pressure measurement chamber (20) comprises at least two compartments (22, 24), and the sensor material (60) is arranged in a first of the two compartments (22) and the compensation material (70) is arranged in the second of the two compartments (24), wherein the first compartment (22) also comprises a region (30) which is permeable to the analyte and the test medium.

6. The biosensor according to claim 5, **characterised in that** the compartments (22; 24) border one another, and/or **in that** a flexible membrane (40) is arranged between the two compartments (22; 24).

7. The biosensor according to claim 6, **characterised in that** both compartments (22, 24) comprise a region (30, 32) which is permeable to the analyte and the test medium.

8. The biosensor according to any one of claims 5 or 6, **characterised in that** the pressure sensor (56) is arranged at least in part in the second compartment (24) and is surrounded at least in regions by the compensation material (70).

9. The biosensor according to claim 8, **characterised in that** the compensation material (70) is oil (72).

10. The biosensor according to any one of claims 1-4, **characterised in that** the sensor material (60) is mixed in the pressure measurement chamber (20) with the compensation material (70), such that the sensor material (60) and the compensation material (70) are arranged in one compartment (26), and this compartment also comprises a region (30) which is permeable to the analyte and the test medium.

11. The biosensor according to claim 10, **characterised in that** the sensor material (60) together with the compensation material (70) forms a stack arrangement comprising alternating layers of sensor material (60) and compensation material (70), or **in that** a mixture of particles of sensor material (60) and compensation material (70) is provided.

12. The biosensor according to any one of claims 1-7 or 10-11, **characterised in that** the pressure sensor (56) is arranged within the pressure measurement chamber (20) in an additional compartment (50), which can be filled with a pressure transfer medium (54).

13. The biosensor according to any one of claims 1-5, **characterised in that** the first compartment (22) is coupled to the second compartment (24) by a pressure piston connection (80) or by an extendable pipe connection (90), such that a change in volume of the first compartment (22) leads to a change in volume of the second compartment (24).

14. The biosensor according to any one of claims 1-7 or 10-11, **characterised in that** the compensation material (74) is a hydrogel.

15. The biosensor according to any one of the preceding claims, **characterised in that** the biosensor (10) can be implanted in a human or animal body.

16. The biosensor according to any one of claims 1-7, 10-11 or 13-14, **characterised in that** the pressure sensor (56) is connected to the wall of the pressure measurement chamber (20), or one or more components of the pressure sensor (56) is/are part of the wall of the pressure measurement chamber (20).

## Revendications

1. Bio-capteur (10) doté d'un matériau de capteur (60) ayant une sensibilité pour au moins un analyte dans une chambre de mesure de pression (20) couplée avec un capteur de pression (56), où une pression régnant dans la chambre de mesure de pression (20) peut être déterminée par le capteur de pression (56),
**caractérisé en ce que** le matériau de capteur (60) est couplé avec un matériau de compensation (70) dans la chambre de mesure de pression (20)), lequel compense au moins partiellement une modification volumique du matériau de capteur (60) conditionnée par une sensibilité croisée.

2. Bio-capteur selon la revendication 1, **caractérisé en ce que** la modification volumique du matériau de capteur (60) conditionnée par une sensibilité croisée du matériau de capteur (60) est une modification volumique conditionnée par la température et/ou une modification volumique conditionnée par la valeur de pH.

3. Bio-capteur selon la revendication 1 ou 2, **caractérisé en ce que**, dans le cas du matériau de capteur (60) ayant une sensibilité pour au moins un analyte, il s'agit d'un gel de polymère, ou d'un hydrogel ou d'un hydrogel « intelligent » ayant une sensibilité pour au moins un analyte.

4. Bio-capteur selon l'une des revendications 1 à 3, **caractérisé en ce qu'**un effet de volume du matériau de capteur (60) s'ajoute au niveau du matériau de capteur (60) en ce qui concerne, d'une part, une modification de la valeur de pH et/ou une modification de la température et d'autre part, une modification de la concentration de l'analyte.

5. Bio-capteur selon l'une des revendications 1 à 4, **caractérisé en ce que** la chambre de mesure de pression (20) présente au moins deux chambres (22, 24), et le matériau de capteur (60) est disposé dans une première des deux chambres (22) et le matériau de compensation (70) est disposé dans la deuxième des deux chambres (24), où la première chambre (22) présente en outre une région (30) qui est perméable à l'analyte et au milieu de test.

6. Bio-capteur selon la revendication 5, **caractérisé en ce que** les chambres (22 ; 24) sont limitrophes l'une de l'autre et/ou qu'une membrane (40) souple est disposée entre les deux chambres (22 ; 24).

7. Bio-capteur selon la revendication 6, **caractérisé en ce que** les deux chambres (22, 24) présentent une région (30, 32) qui est perméable à l'analyte et au milieu de test.

8. Bio-capteur selon l'une des revendications 5 ou 6, **caractérisé en ce que** le capteur de pression (56) est disposé au moins partiellement dans la deuxième chambre (24) et est entouré au moins partiellement du matériau de compensation (70).

9. Bio-capteur selon la revendication 8, **caractérisé en ce que** le matériau de compensation (70) est de l'huile (72).

10. Bio-capteur selon l'une des revendications 1 à 4, **caractérisé en ce que** le matériau de capteur (60) est mélangé avec le matériau de compensation (70) dans la chambre de mesure de pression (20) de sorte que le matériau de capteur (60) et le matériau de compensation (70) sont disposés dans une chambre (26) et cette chambre présente en outre une région (30) qui est perméable à l'analyte et au milieu de test.

11. Bio-capteur selon la revendication 10, **caractérisé en ce que** le matériau de capteur (60) forme un arrangement empilé avec le matériau de compensation (70) avec des couches alternées de matériau de capteur (60) et de matériau de compensation (70) ou qu'un mélange de particules constituées de matériau de capteur (60) et de matériau de compensation (70) est prévu.

12. Bio-capteur selon l'une des revendications 1 à 7 ou 10 et 11, **caractérisé en ce que** le capteur de pression (56) est disposé à l'intérieur de la chambre de mesure de pression (20) dans une chambre (50) supplémentaire qui peut être remplie avec un milieu de transmission de pression (54).

13. Bio-capteur selon l'une des revendications 1 à 5, **caractérisé en ce que** la première chambre (22) est couplé avec la deuxième chambre (24) par une liaison par piston de pression (80) ou par une liaison tubulaire (90) étirable de telle manière qu'une modification de volume de la première chambre (22) entraîne une modification de volume de la deuxième chambre (24).

14. Bio-capteur selon l'une des revendications 1 à 7 ou 10 et 11, **caractérisé en ce que** le matériau de compensation (74) est un hydrogel.

15. Bio-capteur selon l'une des revendications précédentes, **caractérisé en ce que** le bio-capteur est implantable dans le corps humain ou animal.

16. Bio-capteur selon l'une des revendications 1 à 7, 10 et 11 ou 13 et14, **caractérisé en ce que** le capteur de pression (56) est relié avec la paroi de la chambre de mesure de pression (20) ou un ou plusieurs composants du capteur de pression (56) est (sont) un composant de la paroi de la chambre de mesure de pression (20).
